Europäisches Patentamt

European Patent Office

Office européen des brevets

(11) Publication number: **0 160 269 B1**

(12) **EUROPEAN PATENT SPECIFICATION**

(45) Date of publication of patent specification: **30.10.91** (51) Int. Cl.⁵: **A61K 7/075, C11D 1/94, C11D 1/86, C11D 1/75**

(21) Application number: **85105011.2**

(22) Date of filing: **25.04.85**

(54) Detergent formulations.

(30) Priority: **01.05.84 GB 8411110**

(43) Date of publication of application:
**06.11.85 Bulletin 85/45**

(45) Publication of the grant of the patent:
**30.10.91 Bulletin 91/44**

(84) Designated Contracting States:
**AT BE CH DE FR GB IT LI LU NL SE**

(56) References cited:
**EP-A- 0 071 410        EP-A- 0 164 058**
**FR-A- 1 484 737        FR-A- 2 315 905**
**US-A- 4 329 335        US-A- 4 426 310**

**CHEMICAL ABSTRACTS, vol. 97, no. 14, October 1982, page 333, abstract no. 115159d, Columbus, Ohio, US; & JP-A-82 51 799 (LION CORP.) 26-03-1982**

(73) Proprietor: **BEECHAM GROUP PLC**
**Beecham House Great West Road**
**Brentford Middlesex TW8 9BD(GB)**

(72) Inventor: **Wilkins, Anne Lilian**
**34 Harrowdene Gardens**
**Teddington(GB)**
Inventor: **Stothard, Heather Elizabeth**
**325 Hersham Road Hersham**
**Walton-on-Thames Surrey KT12 5QA(GB)**
Inventor: **Clarkson, Quinten Robert Mark**
**55 Byfleet Road**
**New Haw Weybridge Surrey(GB)**

(74) Representative: **Russell, Brian John et al -**
**Beecham Pharmaceuticals Great Burgh Yew Tree Bottom Road**
**Epsom Surrey KT18 5XQ(GB)**

Rank Xerox (UK) Business Services

**Description**

The present invention relates to detergent formulations and particularly to shampoo formulations which have low irritation properties.

Mild shampoos such as adult frequent use and baby shampoos may be formulated by using certain combinations of materials, some of which synergistically reduce the inherent irritancy of others.

However, there are often formulation difficulties with such combinations due to problems in thickening the final product.

For example, a mild shampoo formulation is known which contains an anionic detergent and an imidazolinium derivative, but this formulation has a low viscosity which is unattractive to the consumer. It has now surprisingly been found that both the viscosity and mildness of the above known type of formulation can be increased by incorporating an amine oxide derivative together with a $C_{1-6}$ alkoxylated dihydric or polyhydric alcohol derivative into the formulation.

US-A-4,426,310 describes a mild shampoo comprising an anionic surfactant and a sulfosuccinate derivative, together with a polyethoxylated glyceryl higher fatty acid monoester.

US-A-4,329,335 describes a medicated shampoo containing, as cleaning and conditioning agent, an amine oxide derivative and, as dispersant, polyoxyethylene sorbitan monolaurate.

US-A-3,711,414 describes an anti-static detergent composition containing an anionic surfactant, an amine oxide and, optionally, an imidazolinium salt.

According to the present invention there is provided a shampoo formulation comprising an anionic surfactant and an imidazolinium derivative or sulphosuccinate derivative, characterised in that the formulation additionally comprises from 0.01 to 5% by weight of an amine oxide derivative and from 0.1 to 10% by weight of a $C_{1-6}$ alkoxylated dihydric or polyhydric alcohol derivative.

The shampoo formulation of the invention is particularly suitable for use as a baby shampoo, due to its mildness. The formulations of the invention also have good foaming and conditioning characteristics.

Suitable amine oxide derivatives for use in the formulation of the invention are alkyl amine oxides or alkyl amidoamine oxides. A preferred amine oxide derivative is cocamidopropylamine oxide marketed by Goldschmidt under the trade name Aminoxid WS 35.

Preferably the formulations contain from 0.1% to 5% by weight of the amine oxide derivative, more preferably from 1% to 3%. A particularly preferred formulation contains about 1.8% by weight of derivative

Preferably, the $C_{1-6}$ alkoxylated dihydric or polyhydric alcohol derivatives are ethoxylated dihydric or polyhydric alcohol derivatives.

Preferred ethoxylated dihydric or polyhydric alcohol derivatives are ethoxylated glyceryl esters, ethoxylated propylene glycol esters and ethoxylated glucose derivatives. A particularly preferred material is ethoxylated methyl glucose dioleate, marketed under the trade name of Glucamate DOE120 by Amerchol International.

Preferably the formulations contain from 2% to 4% by weight of the $C_{1-6}$ alkoxylated dihydric or polyhydric alcohol derivative.

Preferred formulations of the invention containing either an alkyl sulphate or alkyl ether sulphate and an alkyl imidazolinium derivative in the ratios of 80:20 to $\overline{20:80}$ (particularly 50:50) or an alkyl sulphate or alkyl ether sulphate and a monoalkyl sulphosuccinate derivative in the ratio of 80:20 to $\overline{20:80}$ (particularly 50:50).

Examples of alkyl sulphates and alkyl ether sulphates are the $C_{10}$ to $C_{18}$ alkyl sulphates and $C_{10}$ to $C_{18}$ alkyl ether sulphates containing 2 or 3 moles of ethylene oxide. These materials are generally employed in the form of their sodium, potassium, ammonium or mono-, di- or tri-ethanolamine salts.

Particular examples are sodium lauryl sulphate, ammoniun lauryl sulphate, mono-, di- and tri-ethanolamine lauryl sulphates, sodium lauryl ether sulphate (2EO), sodium lauryl ether sulphate (3 EO), potassium lauryl ether sulphate (2 EO), and ammonium lauryl ether sulphate (3 EO).

Further compounds which may be incorporated into the formulation of the present invention include betaines, sulphobetaines, protein hydrolysate derivatives and polymeric conditioning agents.

The shampoo formulations of the invention may also include minor amounts of other ingredients which are commonly employed in shampoos, for example a foam booster, thickener, opacifier, perfume, colouring agent, preservative, protein, and an agent for adjusting pH, the latter usually being in the range 5 to 7.5.

The formulations of the invention can be made by conventional admixture of the various ingredients in the appropriate proportions.

The invention will now be illustrated with reference to the following examples:

EXAMPLE 1

|  | %w/w |
|---|---|
| 3 moles sodium lauryl ether sulphate(SLES) (27.5% active) | 11.0 |
| cocoimidazolinium dicarboxylate (35% active) | 16.0 |
| ethoxylated sorbitan monolaurate | 10.0 |
| cocoamidopropylamine oxide (35% active) | 5.0 |
| ethoxylated methyl glucose dioleate | 4.0 |

EXAMPLE 2

|  | %w/w |
|---|---|
| 3 moles SLES (27.5% active) | 11.0 |
| cocoimidazolinium dicarboxylate (35% active) | 16.0 |
| ethoxylated sorbitan monolaurate | 10.0 |
| ethoxylated propylene glycol mono oleate | 4.0 |
| cocoamidopropylamine oxide (35% active) | 5.0 |

**EXAMPLE 3**

|  | %w/w |
|---|---|
| 3 moles SLES (27.5% active) | 11.0 |
| cocoimidazolinium dicarboxylate (35% active) | 16.0 |
| ethoxylated sorbitan monolaurate | 10.0 |
| ethoxylated glyceryl monotallowate | 4.0 |
| cocoamidopropylamine oxide (35% active) | 5.0 |

**EXAMPLE 4**

|  | %w/w |
|---|---|
| 3 moles SLES (27.5% active) | 11.0 |
| cocoimidazolinium dicarboxylate (35% active) | 16.0 |
| ethoxylated glyceryl monolaurate | 10.0 |
| ethoxylated methyl glucose dioleate | 4.0 |
| cocoamidopropylamine oxide (35% active) | 5.0 |

**EXAMPLE 5**

|  | %w/w |
|---|---|
| 3 moles SLES (27.5% active) | 17.5 |
| disodium salt of ethoxylated laurylamide sulphosuccinic acid monoester (45 % active) | 10.5 |
| ethyoxylated sorbitan monolaurate | 10.0 |
| cocamidopropylamine oxide (35% active) | 5.0 |
| ethoxylated methyl glucose dioleate | 4.0 |

Claims

1. A shampoo formulation comprising an anionic surfactant and an imidazolinium derivative or a sulphosuccinate derivative, characterised in that the formulation additionally comprises from 0.01 to 5% by weight of an amine oxide derivative and from 0.1 to 10% by weight of a $C_{1-6}$ alkoxylated dihydric or polyhydric alcohol derivative.

2. A formulation according to claim 1 in which the amine oxide derivative is an alkyl amine oxide or alkyl amidoamine oxide.

4

3. A formulation according to claim 2 in which the amine oxide derivative is cocamido propylamine oxide.

4. A formulation according to any one of claims 1 to 3 in which the $^{C}$1-6 alkoxylated dihydric or polyhydric alcohol derivative is selected from ethoxylated glyceryl esters, ethoxylated propylene glycol esters and ethoxylated glucose derivatives.

5. A formulation according to any one of claims 1 to 4 containing from 2 to 4% by weight of $^{C}$1-6 alkoxylated dihydric or polyhydric alcohol derivative.

6. A formulation according to any one of claims 1 to 5 containing from 1 to 3% by weight of amine oxide derivative.

7. A formulation according to any one of claims 1 to 6, containing either an alkyl sulphate or alkyl ether sulphate and an alkyl imidazolinium weight ratio of 80:20 to 20:80, or an alkyl sulphate or alkyl ether sulphate and a monoalkyl sulphosuccinate derivative in the weight ration 80:20 to 20:80.

**Revendications**

1. Formulation de shampooing comprenant un tensio-actif anionique et un dérivé d'imidazolinium ou un dérivé sulfosuccinique, caractérisée en ce que la formulation comprend de plus de 0,01 à 5 % en poids d'un dérivé oxyde d'amine et de 0,1 à 10 % en poids d'un dérivé d'alcool dihydrique ou polyhydrique alcoxylé en $C_{1-6}$.

2. Formulation suivant la revendication 1, caractérisée en ce que le dérivé oxyde d'amine est un oxyde d'alkylamine ou un oxyde d'alkylamidoamine

3. Formulation suivant la revendication 2, caractérisée en ce que le dérivé oxyde d'amine est l'oxyde de cocamidopropylamine.

4. Formulation suivant l'une quelconque des revendications 1 à 3, caractérisée en ce que le dérivé d'alcool dihydrique ou polyhydrique alcoxylé en $C_{1-6}$ est choisi parmi des esters glycériliques éthoxylés, des esters de propylèneglycol éthoxylés et des dérivés de glucose éthoxylé.

5. Formulation suivant l'une quelconque des revendications 1 à 4, caractérisée en ce qu'elle contient 2 à 4 % en poids d'un dérivé d'alcool dihydrique ou polyhydrique alcoxylé en $C_{1-6}$.

6. Formulation suivant l'une quelconque des revendications 1 à 5, caractérisée en ce qu'elle contient de 1 à 3 % en poids d'un dérivé oxyde d'amine.

7. Formulation suivant l'une quelconque des revendications 1 à 6, caractérisée en ce qu'elle contient soit un alkylsulfate ou un alkyléthersulfate et un dérivé d'alkylimidazolinium selon un rapport pondéral de 80:20 à 20:80, soit un alkylsulfate ou un alkyléthersulfate et un dérivé monoalkylsulfosuccinate selon un rapport pondéral de 80:20 à 20:80.

**Patentansprüche**

1. Shampooformulierung, die einen anionischen grenzflächenaktiven Stoff und ein Imidazoliniumderivat oder ein Sulfosuccinatderivat umfaßt, **dadurch gekennzeichnet,** daß die Formulierung zusätzlich 0,01 bis 5 Gew.-% eines Aminoxidderivats und 0,1 bis 10 Gew.-% eines $C_{1-6}$-alkoxylierten zwei- oder mehrwertigen Alkoholderivats umfaßt.

2. Formulierung nach Anspruch 1, wobei das Aminoxidderivat ein Alkylaminoxid oder ein Alkylamidoaminoxid ist.

3. Formulierung nach Anspruch 2, wobei das Aminoxidderivat ein Cocamidopropylaminoxid ist.

4. Formulierung nach einem der Ansprüche 1 bis 3, wobei das $C_{1-6}$-alkoxylierte zwei- oder mehrwertige Alkoholderivat ausgewählt ist aus ethoxylierten Glycerinestern, ethoxylierten Propylenglykolestern und ethoxylierten Glucosederivaten.

5. Formulierung nach einem der Ansprüche 1 bis 4, die 2 bis 4 Gew.-% eines $C_{1-6}$-alkoxylierten zwei- oder mehrwertigen Alkoholderivats enthält.

6. Formulierung nach einem der Ansprüche 1 bis 5, die 1 bis 3 Gew.-% eines Aminoxidderivats enthält.

7. Formulierung nach einem der Ansprüche 1 bis 6, die entweder ein Alkylsulfat oder ein Alkylethersulfat und ein Alkylimidazolinium in einem Gewichtsverhältnis von 80:20 bis 20:80, oder ein Alkylsulfat oder Alkylethersulfat und ein Monoalkylsulfosuccinatderivat in einem Gewichtsverhältnis von 80:20 bis 20:80 enthält.